# FASCICULE DE BREVET EUROPEEN

(11) **EP 1 223 913 B1**
(45) Date de publication et mention de la délivrance du brevet: **08.03.2006**
(21) Numéro de dépôt: 00990098.6
(22) Date de dépôt: 21.12.2000
(51) Int. Cl.: A61Q 19/00, A61K 8/368, A61K 8/44, A61K 31/12, A61K 31/13, A61K 31/185, A61K 31/21, A61K 31/352, A61K 31/357, A61K 31/382, A61K 31/39, A61K 31/435, A61K 31/498, A61P 17/00, A61P 3/04

(54) **UTILISATION DE COMPOSES POLYCYCLIQUES AROMATIQUES EN TANT QU'ACTIVATEURS DES RECEPTEURS DE TYPE PPARs DANS UNE COMPOSITION COSMETIQUE OU PHARMACEUTIQUE**
VERWENDUNG VON POLYZYKLISCHEN AROMATISCHEN VERBINDUNGEN ALS PPARS-REZEPTOREN-AKTIVATOREN IN KOSMETISCHEN UND PHARMAZEUTISCHEN ZUSAMMENSETZUNGEN
USE OF AROMATIC POLYCYCLIC COMPOUNDS AS ACTIVATORS OF PPARS-TYPE RECEPTORS IN A COSMETIC OR PHARMACEUTICAL COMPOSITION

(30) Priorité: 22.12.1999 FR 9916270
(43) Date de publication de la demande: 24.07.2002
(73) Titulaire: Galderma Research & Development, 06560 Valbonne (FR)
(72) Inventeur: BERNARDON, Jean-Michel, F-06650 Le Rouret (FR); MICHEL, Serge, F-06330 Roquefort-les-Pins (FR)
(74) Mandataire: Renard, Emmanuelle
(86) Numéro de dépôt international: PCT/FR2000/003646
(87) Numéro de publication internationale: WO 2001/045664

(56) Documents cités:
- US-A- 4 829 080
- US-A- 5 004 732
- US-A- 5 763 487

## Description

La présente invention concerne une utilisation de composés pour la fabrication d'une composition pharmaceutique destinée à traiter des désordres cutanés de la fonction barrière et plus particulièrement les désordres de la sécrétion des lipides épidermiques, les photo-dermatoses, ou des ulcères ; et/ou des troubles du métabolisme des lipides.

La présente invention concerne également un procédé non thérapeutique de traitement cosmétique pour restaurer la fonction barrière et plus particulièrement pour réguler le métabolisme des lipides cutanés caractérisé en ce qu'on applique sur la peau une composition comprenant au moins un composé de formule (I), et plus particulièrement en tant qu'activateur des récepteurs de type PPARs.

De manière tout à fait surprenante et inattendue, la Demanderesse a trouvé que certains composés décrits dans les demandes de brevet EP 722 928 comme composés ayant une action antiproliférative ont une activité marquée vis à vis de la transactivation des récepteurs de type PPARs.

Cette découverte est à la base de la présente invention.

La présente invention a donc trait à une utilisation d'une quantité efficace d'au moins un composé, et plus particulièrement en tant qu'activateurs des récepteurs de type PPARs, pour la fabrication d'une composition pharmaceutique destinée à traiter des désordres cutanés de la fonction barrière et plus particulièrement les désordres de la sécrétion des lipides épidermiques, les photo-dermatoses, ou des ulcères; et/ou des troubles du métabolisme des lipides.

La présente invention concerne également un procédé non thérapeutique de traitement cosmétique pour restaurer la fonction barrière et plus particulièrement pour réguler le métabolisme des lipides cutanés caractérisé en ce qu'on applique sur la peau une composition comprenant au moins un composé de formule (I), et plus particulièrement en tant qu'activateur des récepteurs de type PPARs.

Ces composés présentent une formule générale (I): 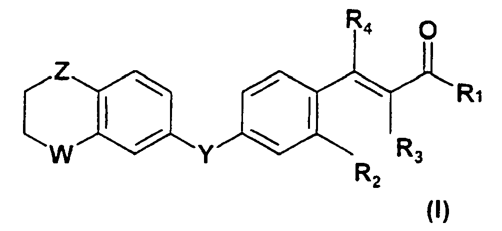
dans laquelle
R₁ représente un atome d'hydrogène ou un radical -OR₅
R₅ ayant les significations données ci-après,
R₂ représente un atome d'hydrogène ou un radical alkyle inférieur,
R₃ et R₄ identiques ou différents représentent un atome d'hydrogène, ou un radical alkyle inférieur,
étant entendu que R₂ et R₃ pris ensemble peuvent former avec le cycle benzénique adjacent un cycle naphthalénique,
Y représente un atome d'oxygène, un radical S(O)ₙ, ou un radical N-R₆
n et R₆ ayant les significations données ci-après,
Z et W, identiques ou différents, représentent -CR₇R₈-, -O-, ou -S(O)ₘ,
m, R₇ et R₈ ayant les significations données ci-après,
R₅ représente un atome d'hydrogène, un radical alkyle linéaire ou ramifié ayant de 1 à 20 atomes de carbone, ou un radical mono ou polyhydroxyalkyle,
R₅ représente un atome d'hydrogène, ou un radical alkyle inférieur,
R₇ et R₈, identiques ou différents, représentent un atome d'hydrogène ou un radical alkyle inférieur,
n représente 0, 1 ou 2,
m représente 0, 1 ou 2,
ainsi que leurs sels, et leurs analogues chiraux. Les composés de formule (I) peuvent se présenter sous forme de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Parmi les radicaux alkyle linéaire ou ramifié ayant de 1 à 20 atomes de carbone, on peut notamment citer les radicaux méthyle, éthyle, propyle, 2-éthyl-hexyle, octyle, dodécyle, hexadécyle et octadécyle.

Par radical monohydroxyalkyle, on entend un radical ayant 1 à 6 atomes de carbone et de préférence ayant de 2 à 3 atomes de carbone, notamment un radical 2-hydroxyéthyle, 2-hydroxypropyle ou 3-hydroxypropyle.

Par radical polyhydroxyalkyle, on entend un radical contenant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les composés de formule (I) ci-dessus, on peut notamment citer les suivants:
Composé 1 : acide 6-(5,5,8,8-Tétraméthyle-5,6,7,8-tétrahydro-naphthalene-2-yloxy)-naphthalene-2-carboxylique
Composé 2 : acide 3-[4-(5,5,8,8-Tétraméthyle-5,6,7,8-tétrahydro-naphthalene-2-yloxy)-phényl]-acrylique
Composé 3 : acide 6-(5,5,8,8-Tétraméthyle-5,6,7,8-tétrahydro-naphthalene-2-ylsulfanyl)-naphthalene-2-carboxylique
Composé 4 : acide 3-[4-(5,5,8,8-Tétraméthyle-5,6,7,8-tétrahydro-naphthalene-2-yloxy)-phényl]-but-2-énoïque
Composé 5 : acide 6-(5,5,8,8-Tétraméthyle-5,6,7,8-tétrahydro-naphthalene-2-ylamino)-naphthalene-2-carboxylique

Selon la présente invention le composé de formule (I) plus particulièrement utilisé est le composé 3 : l'acide 6-(5,5,8,8-Tétraméthyle-5,6,7,8-tétrahydro-naphthalene-2-ylsulfanyl)-naphthalene-2-carboxylique.

Les composés de formule (I) peuvent être notamment obtenus par les procédés de préparation décrits dans la demande de brevet EP 722 928.

Les composés de l'invention présentent des propriétés d'activation des récepteurs de type PPARs. Les récepteurs de type PPARs sont des récepteurs qui appartiennent à la famille des récepteurs nucléaires stéroïdiens.

Par activateur des récepteurs de type PPARs, on entend selon l'invention tout composé qui présente dans un test de transactivation, tel que décrit dans Kliewer et al., Nature 358, 771-774, 1992, une AC50 inférieure ou égale à 10 µM. De préférence, l'activateur des récepteurs de type PPAR présente une AC50 inférieure ou égale à 2 µM et avantageusement inférieure ou égale à 1 µM.

Une AC50 est la concentration en composé "activateur" nécessaire pour présenter 50% de l'activité d'une molécule de référence. Cette activité est déterminée à l'aide d'une enzyme (luciférase) rapporteuse de l'activation due au composé via un des récepteurs PPARs.

L'activité des récepteurs de type PPARs a fait l'objet de nombreuses études. On peut citer à titre indicatif la publication intitulée "Differential Expression of Peroxisome Proliferator-Activated Receptor Subtypes During the Differentiation of Human Keratinocytes", Michel Rivier et al., J. Invest. Dermatol 111, 1998, p 1116-1121, dans laquelle sont répertoriées un grand nombre de références bibliographiques concernant les récepteurs de type PPARs.

L'utilisation des activateurs des récepteurs de type PPAR-α pour restaurer la fonction barrière et plus particulièrement pour traiter les désordres de la sécrétion des lipides épidermiques, promouvoir la différentiation et inhiber la prolifération épidermique a été décrite dans la demande de brevet internationale WO 98/32444.

De plus, l'utilisation des activateurs des récepteurs de type PPAR-α et/ou PPAR-γ pour traiter les désordres cutanés liés à une anomalie de la différenciation des cellules épidermiques a été décrite dans la publication de Michel Rivier et al., J. Invest. Dermatol 111, 1998, p 1116-1121.

Il a également été décrit dans la demande de brevet WO 96/33724 que des composés sélectifs des PPARγ, tels qu'une prostaglandine-J2 ou -D2 sont des actifs potentiels pour le traitement de l'obésité et du diabète.

Les compositions pharmaceutiques contenant au moins un composé de formule (I) sont donc destinées au traitement des désordres cutanés de la fonction barrière et plus particulièrement les désordres de la sécrétion des lipides épidermiques, les photo-dermatoses ou des ulcères; et/ou des troubles du métabolisme des lipides.

Parmi les désordres de la fonction barrière et plus particulièrement les désordres de la sécrétion des lipides épidermiques on peut citer notamment, les désordres de la peau des enfants prématurés nés avant 33 semaines, les fissures labiales, ou les bulles suite à friction mécanique.

Parmi les ulcères, on peut citer notamment les ulcères et érosions dus à des brûlures chimiques ou thermiques, les désordres bulleux, ou les troubles vasculaires ou ischémie incluant les ulcères veineux, artériels, emboliques ou diabétiques.

Parmi les affections du métabolisme des lipides, on peut citer l'obésité, l'hyperlipidémie, ou le diabète non insulino-dépendant.

L'administration de la composition selon l'invention peut être effectuée par voie entérale, parentérale, topique, ou oculaire. De préférence, la composition pharmaceutique est conditionnée sous une forme convenant à une application par voie topique.

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les composés utilisés selon l'invention sont généralement administrés à une dose journalière d'environ 0,001 mg/kg à 100 mg/kg en poids corporel en 1 à 3 prises.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter soit sous forme anhydre, soit sous forme aqueuse.

Les composés sont utilisés par voie topique à une concentration généralement comprise entre 0,001 % et 10 % en poids, de préférence entre 0,01 et 1 % en poids, par rapport au poids total de la composition.

Les composés de formule (I) selon l'invention trouvent également une application dans le domaine cosmétique, en particulier dans l'hygiène corporelle et capillaire et plus particulièrement pour restaurer la fonction barrière et plus particulièrement pour réguler et/ou restaurer le métabolisme des lipides cutanés. Par rapport aux produits connus antérieurement, ces composés de formule (I) ont l'avantage de présenter en plus d'autres propriétés intéressantes, notamment des propriétés anti-inflammatoires ou apaisantes, ce qui en fait des composés moins irritants et donc mieux tolérés.

La composition cosmétique selon l'invention contenant, dans un support cosmétiquement acceptable, au moins un composé de formule (I) l'un de ses isomères optiques ou géométriques ou l'un de ses sels, peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques est comprise entre 0,001 et 3 % en poids.

D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

Les compositions telles que décrites précédemment peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; les acides eicosa-5,8,11,14-tétraynoïque et eicosa-5,8,11-triynoïque, leurs esters et amides et enfin les rétinoïdes. Les composés de formule (I) peuvent également être combinés avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre d'illustration et sans aucun caractère limitatif, plusieurs exemples de résultats de tests biologiques de composés actifs de formule (I) selon l'invention, ainsi que diverses formulations concrètes à base de tels composés. Dans ce qui suit ou ce qui précède, les pourcentages sont donnés en poids sauf mention contraire.

### EXEMPLE 1

Dans cet exemple, on a illustré divers résultats de tests biologiques qui montrent les propriétés de transactivation des récepteurs PPARs des composés de l'invention.

Les exemples comparatifs correspondent à des composés qui sont décrits dans la demande de brevet EP 722 928 mais qui ne vérifient pas les conditions des composés de formule (I).

Les tests biologiques effectués correspondent à ceux décrits dans la demande. La méthode utilisée pour déterminer les AC50 est celle décrite dans Kliewer et al., Nature 358, 771-774, 1992. Ainsi, le pouvoir activateur via PPAR-α, PPAR-γ ou PPAR-δ, de molécules peut être évalué avec un test de transactivation dans lequel les cellules HeLa ont été cotransfectées par un vecteur d'expression codant pour ces récepteurs et un plasmide rapporteur contenant un élément de réponse PPRE cloné en amont d'une partie d'un promoteur du virus SV40 et du gène luciférase. Les cellules cotransfectées sont traitées pendant 24 heures avec les molécules à tester et l'activité de la luciférase est déterminée par luminescence.

La référence 1, molécule de référence des PPAR-α est l'acide [4-Chloro-6(2.3-diméthyle-phénylamino)-pyrimidin-2-ylsulfanyl]acétique;
La référence 2, molécule de référence des PPAR-δ et PPAR-γ est la 5-{4[2-(méthyle-pyridin-2-yl-amino)-éthoxy]-benzyl}-thiazolidine-2,4-dione;

L'exemple comparatif 1 est le 2-Méthyle-4-[4-(5,5,8,8-tétraméthyle-5,6,7,8-tétrahydro-naphthalene-2-yloxy)-benzylidène]-4H-oxazol-5-one.
L'exemple comparatif 2 est l'acide 2-Acétylamino-3-[4-(5,5,8,8-tétraméthyle-5,6,7,8-tétrahydro-naphthalene-2-yloxy)-phényl]-acrylique.

Les résultats obtenus dans les tests de transactivation des récepteurs de type PPARs sont regroupés dans le tableau suivant :

| composés | α | γ | β |
|---|---|---|---|
| Référence 1 | 100*(1,4)** | n.a | n.a |
| Référence 2 | n.a | 100(0,07) | 100(0,13) |
| Composé 1 | 18 | 23 | 152 (0,7) |
| Composé 2 | 12 | 18 | 204 (0,9) |
| Composé 3 | 24 | 40 | 172 (0,2) |
| Composé 4 | 12 | 0 | 56 |
| Composé 5 | 25 | 69 | 328 (7) |
| Exemple comparatif 1 | 5 | 0 | 7 |
| Exemple comparatif 2 | 7 | 4 | 0 |

| | | | |
|---|---|---|---|
| n.a signifie non actif | | | |
| * % d'activation | | | |
| ()** AC₅₀ en µM | | | |

Ces résultats montrent l'activation des composés de l'invention pour les différents sous-types de récepteurs de type PPARs : PPAR-α, PPAR-β, et PPAR-γ.

### EXEMPLE 2

Dans cet exemple, on a illustré diverses formulations concrètes à base des composés selon l'invention.

### A- VOIE ORALE

### (a) Comprimé de 0,2 g

- Composé 1 0,001 g
- Amidon 0,114 g
- Phosphate bicalcique 0,020 g
- Silice 0,020 g
- Lactose 0,030 g
- Talc 0,010 g
- Stéarate de magnésium 0,005 g

### (b) Suspension buvable en ampoules de 5 ml

- Composé 5 0,001 g
- Glycérine 0,500 g
- Sorbitol à 70% 0,500 g
- Saccharinate de sodium 0,010 g
- Parahydroxybenzoate de méthyle 0,040 g
- Arome qs
- Eau purifiée qsp 5 ml

### (c) Comprimé de 0,8 g

- Composé 2 0,500 g
- Amidon prégélatinisé 0,100 g
- Cellulose microcristalline 0,115 g
- Lactose 0,075 g
- Stéarate de magnésium 0,010 g

### (d) Suspension buvable en ampoules de 10 ml

- Composé 4 0,200 g
- Glycérine 1,000 g
- Sorbitol à 70% 1,000 g
- Saccharinate de sodium 0,010 g
- Parahydroxybenzoate de méthyle 0.080 g
- Arome qs
- Eau purifiée qsp 10 ml

### B- VOIE TOPIQUE

### (a) Onguent

- Composé 1 0,020 g
- Myristate d'isopropyle 81,700 g
- Huile de vaseline fluide 9,100 g
- Silice ("Aérosil 200" vendue par DEGUSSA) 9,180 g

### (b) Onguent

- Composé 2 0,300 g
- Vaseline blanche codex qsp 100 g

### (c) Crème Eau-dans-Huile non ionique

- Composé 1 0,100 g
- Mélange d'alcools de lanoline émulsifs, de cires et d'huiles ("Eucerine anhydre" vendu par BDF) 39,900 g
- Parahydroxybenzoate de méthyle 0,075 g
- Parahydroxybenzoate de propyle 0,075 g
- Eau déminéralisée stérile qsp 100 g

### (d) Lotion

- Composé 3 0,100 g
- Polyéthylène glycol (PEG 400) 69,900 g
- Ethanol à 95% 30,000 g

### (e) Onguent hydrophobe

- Composé 5 0,300 g
- Miristate d'isopropyle 36,400 g
- Huile de silicone ("Rhodorsil 47 V 300" vendu par RHONE-POULENC) 36,400 g
- Cire d'abeille 13,600 g
- Huile de silicone ("Abil 300.000 cst" vendu par GOLDSCHMIDT) qsp 100 g

### (f) Crème Huile-dans-Eau non ionique

- Composé 2 1,000 g
- Alcool cétylique 4,000 g
- Monostéarate de glycérole 2,500 g
- Stéarate de PEG 50 2,500 g
- Beurre de karité 9,200 g
- Propylène glycol 2,000 g
- Parahydroxybenzoate de méthyle 0,075 g
- Parahydroxybenzoate de propyle 0.075 g
- Eau déminéralisée stérile qsp 100 g

## Revendications

1. Utilisation d'une quantité efficace d'au moins un composé de formule (I) pour la fabrication d'une composition pharmaceutique destinée à traiter des désordres cutanés de la fonction barrière et plus particulièrement les désordres de la sécrétion des lipides épidermiques, les photo-dermatoses ou des ulcères ; et/ou des troubles du métabolisme des lipides, ce composé présentant une formule générale (I) : 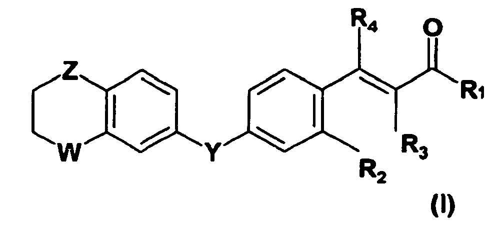
dans laquelle
R₁ représente un atome d'hydrogène ou un radical -OR₅
R₅ ayant les significations données ci-après,
R₂ représente un atome d'hydrogène ou un radical alkyle inférieur,
R₃ et R₄ identiques ou différents représentent un atome d'hydrogène, ou un radical alkyle inférieur,
étant entendu que R₂ et R₃ pris ensemble peuvent former avec le cycle benzénique adjacent un cycle naphthalénique,
Y représente un atome d'oxygène, un radical S(O)ₙ, ou un radical N-R₆
n et R₆ ayant les significations données ci-après,
Z et W, identiques ou différents, représentent -CR₇R₈-, -O-, ou -S(O)ₘ,
m, R₇ et R₈ ayant les significations données ci-après,
R₅ représente un atome d'hydrogène, un radical alkyle linéaire ou ramifié ayant de 1 à 20 atomes de carbone, ou un radical mono ou polyhydroxyalkyle,
R₆ représente un atome d'hydrogène, ou un radical alkyle inférieur,
R₇ et R₈ identiques ou différents représentent un atome d'hydrogène, ou un radical alkyle inférieur,
n représente 0, 1 ou 2,
m représente 0, 1 ou 2,
ainsi que leurs sels, et leurs analogues chiraux.

2. Utilisation selon la revendication 1, **caractérisée en ce que** les composés de formule (I) sont utilisés en tant qu'activateurs des récepteurs de type PPARs.

3. Utilisation selon l'une quelconque des revendications 1 ou 2, **caractérisée en ce que** les composés de formule (I) se présentent sous forme de sels d'un métal alcalin ou alcalino-terreux, ou encore de zinc ou d'une amine organique.

4. Utilisation selon l'une quelconque des revendications 1 à 3, **caractérisée en ce que** les radicaux alkyles inférieurs sont choisis dans le groupe constitué par les radicaux méthyle, éthyle, isopropyle, butyle, tertiobutyle et hexyle.

5. Utilisation selon l'une quelconque des revendications précédentes, **caractérisée en ce que** les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

6. Utilisation selon l'une quelconque des revendications précédentes, **caractérisée en ce que** les radicaux monohydroxyalkyles sont choisis dans le groupe constitué par les radicaux 2-hydroxyéthyle, 2-hydroxypropyle ou 3-hydroxypropyle.

7. Utilisation selon l'une quelconque des revendications précédentes, **caractérisée en ce que** les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

8. Utilisation selon l'une quelconque des revendications 1 ou 2, **caractérisée par le fait que** les composés sont choisis dans le groupe constitué par :
Composé 1 : acide 6-(5,5,8,8-Tétraméthyle-5,6,7,8-tétrahydro-naphthalene-2-yloxy)-naphthalene-2-carboxylique
Composé 2 : acide 3-[4-(5,5,8,8-Tétraméthyle-5,6,7,8-tétrahydro-naphthalene-2-yloxy)-phényl]-acrylique
Composé 3 : acide 6-(5,5,8,8-Tétraméthyle-5,6,7,8-tétrahydro-naphthalene-2-ylsulfanyl)-naphthalene-2-carboxylique
Composé 4 : acide 3-[4-(5,5,8,8-Tétraméthyle-5,6,7,8-tétrahydro-naphthalene-2-yloxy)-phényl]-but-2-énoïque
Composé 5 : acide 6-(5,5,8,8-Tétraméthyle-5,6,7,8-tétrahydro-naphthalene-2-ylamino)-naphthalene-2-carboxylique

9. Utilisation selon l'une quelconque des revendications 1 ou 2, **caractérisée en ce que** le composé correspondant à la formule (I) est l'acide 6-(5,5,8,8-tétraméthyle-5,6,7,8-tétrahydro-naphthalene-2-ylsulfanyl)-naphthalene-2-carboxylique.

10. Utilisation selon l'une quelconque des revendications 1 ou 2, **caractérisée en ce que** les désordres de la fonction barrière et plus particulièrement les désordres de la sécrétion des lipides épidermiques sont les désordres de la peau des enfants prématurés nés avant 33 semaines, les fissures labiales, ou les bulles suite à friction mécanique.

11. Utilisation selon l'une quelconque des revendications 1 ou 2, **caractérisée en ce que** les ulcères sont les ulcères et érosions dus à des brûlures chimiques ou thermiques, les désordres bulleux, ou les troubles vasculaires ou ischémie incluant les ulcères veineux, artériels, emboliques ou diabétiques.

12. Utilisation selon l'une quelconque des revendications 1 ou 2, **caractérisée en ce que** les troubles du métabolisme des lipides sont l'obésité, l'hyperlipidémie, ou le diabète non insulino-dépendant.

13. Utilisation selon l'une quelconque des revendications précédentes, **caractérisée en ce que** les composés de formule (I) sont combinés avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

14. Utilisation selon l'une quelconque des revendications précédentes, **caractérisée en ce que** la composition est administrée par voie entérale ou parentérale.

15. Utilisation selon l'une quelconque des revendications 1 à 13, **caractérisée en ce que** la composition est administrée par voie topique ou oculaire.

16. Utilisation selon la revendication précédente, **caractérisée en ce que** les composés de formule (I) sont utilisés à une concentration comprise entre 0,001% et 10% en poids par rapport au poids total de la composition.

17. Procédé non thérapeutique de traitement cosmétique pour restaurer la fonction barrière et plus particulièrement pour réguler le métabolisme des lipides cutanés **caractérisé en ce qu'**on applique sur la peau une composition comprenant au moins un composé de formule (I) dans une quantité efficace.

18. Procédé non thérapeutique de traitement cosmétique pour restaurer la fonction barrière et plus particulièrement pour réguler le métabolisme des lipides cutanés **caractérisé en ce qu'**on applique sur la peau une composition comprenant au moins un composé de formule (I) en tant qu'activateur des récepteurs de type PPARs dans une quantité efficace.

19. Procédé non thérapeutique de traitement cosmétique selon l'une quelconque des revendications 17 ou 18, **caractérisée en ce que** la concentration en composés de formule (I) est comprise entre 0,001% et 3% en poids par rapport à l'ensemble de la composition.

## Patentansprüche

1. Verwendung einer wirksamen Menge mindestens einer Verbindung der Formel (I) für die Herstellung einer pharmazeutischen Zusammensetzung, die für die Behandlung von Störungen der Barrierefunktion der Haut und insbesondere Störungen der Sekretion von epidermalen Lipiden, Photodermatosen oder Geschwüren; und/oder Störungen des Metabolismus von Lipiden vorgesehen ist, wobei die Verbindung die folgende allgemeine Formel (I) aufweist: 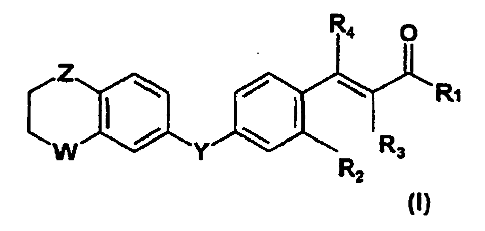
worin bedeuten:
R₁ ein Wasserstoffatom oder eine Gruppe -OR₅,
wobei R₅ die unten angegebenen Bedeutungen aufweist,
R₂ ein Wasserstoffatom oder eine niedere Alkylgruppe,
R₃ und R₄, die gleich oder verschieden sind, ein Wasserstoffatom oder eine niedere Alkylgruppe,
mit der Maßgabe, dass R₂ und R₃ gemeinsam mit dem angrenzenden Benzolring einen Naphthalinring bilden können,
Y ein Sauerstoffatom, eine Gruppe S(O)ₙ oder N-R₆,
wobei n und R₆ die unten angegebenen Bedeutungen aufweisen,
Z und W, die gleich oder verschieden sind, -CR₇R₈-, -O- oder -S(O)m,
wobei m, R₇ und R₈ die unten angegebenen Bedeutungen aufweisen,
R₅ ein Wasserstoffatom, eine geradkettige oder verzweigte Alkylgruppe mit 1 bis 20 Kohlenstoffatomen oder eine Mono- oder Polyhydroxyalkylgruppe,
R₆ ein Wasserstoffatom oder eine niedere Alkylgruppe,
R₇ und R₈, die gleich oder verschieden sind, ein Wasserstoffatom oder eine niedere Alkylgruppe,
n 0, 1 oder 2,
m 0, 1 oder 2,
sowie deren Salze und chiralen Analoga.

2. Verwendung nach Anspruch 1, **dadurch gekennzeichnet, dass** die Verbindungen der Formel (I) als Aktivatoren der Rezeptoren vom Typ der PPARs verwendet werden.

3. Verwendung nach einem der Ansprüche 1 oder 2, **dadurch gekennzeichnet, dass** die Verbindungen der Formel (I) in Form der Salze mit einem Alkali- oder Erdalkalimetall, als Zinksalze oder Salze mit einem organischen Amin vorliegen.

4. Verwendung nach einem der Ansprüche 1 bis 3, **dadurch gekennzeichnet, dass** die niederen Alkylgruppen unter Methyl, Ethyl, Isopropyl, Butyl, *t*-Butyl und Hexyl ausgewählt sind.

5. Verwendung nach einem der vorhergehenden Ansprüche, **dadurch gekennzeichnet, dass** die geradkettigen oder verzweigten Alkylgruppen mit 1 bis 20 Kohlenstoffatomen unter den Gruppen Methyl, Ethyl, Propyl, 2-Ethylhexyl, Octyl, Dodecyl, Hexadecyl und Octadecyl ausgewählt sind.

6. Verwendung nach einem der vorhergehenden Ansprüche, **dadurch gekennzeichnet, dass** die Monohydroxyalkylgruppen unter 2-Hydroxyethyl, 2-Hydroxypropyl oder 3-Hydroxypropyl ausgewählt sind.

7. Verwendung nach einem der vorhergehenden Ansprüche, **dadurch gekennzeichnet, dass** die Polyhydroxyalkylgruppen unter 2,3-Dihydroxypropyl, 2,3,4-Trihydroxybutyl, 2,3,4,5-Tetrahydroxypentyl oder Pentaerythrit ausgewählt sind.

8. Verwendung nach einem der Ansprüche 1 oder 2, **dadurch gekennzeichnet, dass** die Verbindungen ausgewählt sind unter:
Verbindung 1: 6-(5,5,8,8-Tetramethyl-5,6,7,8-tetrahydronaphthalin-2-yloxy)-naphthalin-2-carbonsäure,
Verbindung 2: 3-[4-(5,5,8,8-Tetramethyl-5,6,7,8-tetrahydronaphthalin-2-yloxy)phenyl]-acrylsäure,
Verbindung 3: 6-(5,5,8,8-Tetramethyl-5,6,7,8-tetrahydronaphthalin-2-ylsulfanyl)-naphthalin-2-carbonsäure,
Verbindung 4: 3-[4-(5,5,8,8-Tetramethyl-5,6,7,8-tetrahydronaphthalin-2-yloxy)phenyl]-but-2-ensäure,
Verbindung 5: 6-(5,5,8,8-Tetramethyl-5,6,7,8-tetrahydronaphthalin-2-ylamino)-naphthalin-2-carbonsäure.

9. Verwendung nach einem der Ansprüche 1 oder 2, **dadurch gekennzeichnet, dass** die Verbindung, die der Formel (I) entspricht, die 6-(5,5,8,8-Tetramethyl-5,6,7,8-tetrahydronaphthalin-2-ylsulfonyl)-naphthalin-2-carbonsäure ist.

10. Verwendung nach einem der Ansprüche 1 oder 2, **dadurch gekennzeichnet, dass** die Störungen der Barrierefunktion und insbesondere die Störungen der Sekretion von epidermalen Lipiden Hautstörungen bei Frühgeburten, die vor 33 Wochen geboren sind, labialen Rissbildungen oder Blasen nach mechanischer Reibung ausgewählt sind.

11. Verwendung nach einem der Ansprüche 1 oder 2, **dadurch gekennzeichnet, dass** die Geschwüre unter den Geschwüren und Abschürfungen, die durch chemische oder thermische Verbrennungen verursacht werden, bullösen Störungen oder vaskulären Störungen oder Ischämie, einschließlich venösen, arteriellen, embolischen oder diabetischen Geschwüren ausgewählt sind.

12. Verwendung nach einem der Ansprüche 1 oder 2, **dadurch gekennzeichnet, dass** es sich bei den Störungen des Metabolismus der Lipide um Adipositas, Hyperlipidämie oder nichtinsulinpflichtigen Diabetes handelt.

13. Verwendung nach einem der vorhergehenden Ansprüche, **dadurch gekennzeichnet, dass** die Verbindungen der Formel (I) mit weiteren Verbindungen mit einer Aktivität vom Retinoidtyp, mit D-Vitaminen oder deren Derivaten, mit Corticosteroiden, mit Radikalfängern für freie Radikale, mit α-Hydroxysäuren oder α-Ketosäuren oder deren Derivaten oder Ionenkanalblockern kombiniert werden.

14. Verwendung nach einem der vorhergehenden Ansprüche, **dadurch gekennzeichnet, dass** die Zusammensetzung auf enteralem oder parenteralem Weg verabreicht wird.

15. Verwendung nach einem der Ansprüche 1 bis 13, **dadurch gekennzeichnet, dass** die Zusammensetzung auf topischem oder okularem Weg verabreicht wird.

16. Verwendung nach dem vorhergehenden Anspruch, **dadurch gekennzeichnet, dass** die Verbindung der Formel (I) in einer Konzentration von 0,001 bis 10 Gew.-%, bezogen auf das Gesamtgewicht der Zusammensetzung, verwendet wird.

17. Nichttherapeutisches Verfahren zur kosmetischen Behandlung, um die Barrierefunktionen wiederherzustellen und insbesondere den Metabolismus der Lipide der Haut zu regulieren, **dadurch gekennzeichnet, dass** auf die Haut eine Zusammensetzung aufgetragen wird, die mindestens eine Verbindung der Formel (I) in einer wirksamen Menge enthält.

18. Nichttherapeutisches Verfahren zur kosmetischen Behandlung, um die Barrierefunktionen wiederherzustellen und insbesondere den Metabolismus der Lipide der Haut zu regulieren, **dadurch gekennzeichnet, dass** auf die Haut eine Zusammensetzung aufgetragen wird, die mindestens eine Verbindung der Formel (I) als Aktivator der Rezeptoren vom Typ der PPARs in einer wirksamen Menge enthält.

19. Nichttherapeutisches Verfahren zur kosmetischen Behandlung nach einem der Ansprüche 17 oder 18, **dadurch gekennzeichnet, dass** die Konzentration der Verbindungen der Formel (I) im Bereich von 0,001 bis 3 Gew.-%, bezogen auf die gesamte Zusammensetzung, liegt.

## Claims

1. Use of an effective amount of at least one compound of formula (I) in the manufacture of a pharmaceutical composition intended for the treatment of cutaneous disorders of the barrier function and more particularly disorders of the secretion of epidermal lipids, photodermatoses or ulcers, and/or disorders of the metabolism of lipids, this compound having the general formula (I) : 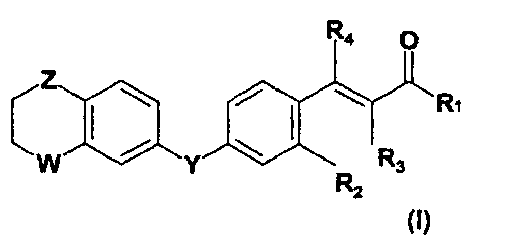
in which
R₁ represents a hydrogen atom or an -OR₅ radical, R₅ having the meanings given below,
R₂ represents a hydrogen atom or a lower alkyl radical,
R₃ and R₄, which are identical or different, represent a hydrogen atom or a lower alkyl radical,
it being understood that R₂ and R₃, taken together, can form a naphthalene ring with the adjacent benzene ring,
Y represents an oxygen atom, an S(O)ₙ radical or an N-R₆ radical,
n and R₆ having the meanings given below,
Z and W, which are identical or different, represent -CR₇R₈-, -O- or -S(O)ₘ,
m, R₇ and R₈ having the meanings given below,
R₅ represents a hydrogen atom, a linear or branched alkyl radical having from 1 to 20 carbon atoms or a mono- or polyhydroxyalkyl radical,
R₆ represents a hydrogen atom or a lower alkyl radical,
R₇ and R₈, which are identical or different, represent a hydrogen atom or a lower alkyl radical,
n represents 0, 1 or 2,
m represents 0, 1 or 2,
and their salts and their chiral analogues.

2. Use according to Claim 1, **characterized in that** the compounds of formula (I) are used as activators of receptors of PPAR type.

3. Use according to either one of Claims 1 and 2, **characterized in that** the compounds of formula (I) are provided in the form of salts of an alkali metal or alkaline-earth metal or of zinc or of an organic amine.

4. Use according to any one of Claims 1 to 3, **characterized in that** the lower alkyl radicals are chosen from the group consisting of the methyl, ethyl, isopropyl, butyl, tert-butyl and hexyl radicals.

5. Use according to any one of the preceding claims, **characterized in that** the linear or branched alkyl radicals having from 1 to 20 carbon atoms are chosen from the group consisting of the methyl, ethyl, propyl, 2-ethylhexyl, octyl, dodecyl, hexadecyl and octadecyl radicals.

6. Use according to any one of the preceding claims, **characterized in that** the monohydroxyalkyl radicals are chosen from the group consisting of the 2-hydroxyethyl, 2-hydroxypropyl and 3-hydroxypropyl radicals.

7. Use according to any one of the preceding claims, **characterized in that** the polyhydroxyalkyl radicals are chosen from the group consisting of the 2,3-dihydroxypropyl, 2,3,4-trihydroxybutyl and 2,3,4,5-tetrahydroxypentyl radicals and the pentaerythritol residue.

8. Use according to either one of Claims 1 and 2, **characterized in that** the compounds are chosen from the group consisting of:
Compound 1: 6-(5,5,8,8-tetramethyl-5,6,7,8-tetrahydronaphth-2-yloxy)naphthalene-2-carboxylic acid
Compound 2: 3-[4-(5,5,8,8-tetramethyl-5,6,7,8-tetrahydronaphth-2-yloxy)phenyl]acrylic acid
Compound 3: 6-(5,5,8,8-tetramethyl-5,6,7,8-tetrahydronaphth-2-ylsulphanyl)naphthalene-2-carboxylic acid
Compound 4: 3-[4-(5,5,8,8-tetramethyl-5,6,7,8-tetrahydronaphth-2-yloxy)phenyl]but-2-enoic acid
Compound 5: 6-(5,5,8,8-tetramethyl-5,6,7,8-tetrahydronaphth-2-ylamino)naphthalene-2-carboxylic acid.

9. Use according to either one of Claims 1 and 2, **characterized in that** the compound corresponding to the formula (I) is 6-(5,5,8,8-tetramethyl-5,6,7,8-tetrahydronaphth-2-ylsulphanyl)naphthalene-2-carboxylic acid.

10. Use according to either one of Claims 1 and 2, **characterized in that** the disorders of the barrier function and more particularly the disorders of the secretion of epidermal lipids are skin disorders in premature babies born before 33 weeks, chapped lips or blisters resulting from mechanical friction.

11. Use according to either one of Claims 1 and 2, **characterized in that** the ulcers are ulcers and erosions due to chemical or thermal burns, bullous disorders or vascular or ischaemia disorders, including venous, arterial, embolic or diabetic ulcers.

12. Use according to either one of Claims 1 and 2, **characterized in that** the disorders of the metabolism of lipids are obesity, hyperlipidaemia or non-insulin-dependent diabetes.

13. Use according to any one of the preceding claims, **characterized in that** the compounds of formula (I) are combined with other compounds possessing activity of retinoid type, with vitamins D or their derivatives, with corticosteroids, with agents for combating free radicals, with α-hydroxy or α-keto acids or their derivatives, or with ion channel blockers.

14. Use according to any one of the preceding claims, **characterized in that** the composition is administered by the enteral or parenteral route.

15. Use according to any one of Claims 1 to 14, **characterized in that** the composition is administered by the topical or ocular route.

16. Use according to the preceding claim, **characterized in that** the compounds of formula (I) are used at a concentration of between 0.001% and 10% by weight with respect to the total weight of the composition.

17. Non-therapeutic cosmetic treatment process for restoring the barrier function and more particularly for regulating the metabolism of cutaneous lipids, **characterized in that** a composition comprising at least one compound of formula (I) in an effective amount is applied to the skin.

18. Non-therapeutic cosmetic treatment process for restoring the barrier function and more particularly for regulating the metabolism of cutaneous lipids, **characterized in that** a composition comprising at least one compound of formula (I), as activator of receptors of PPAR type, in an effective amount is applied to the skin.

19. Non-therapeutic cosmetic treatment process according to either one of Claims 17 and 18, **characterized in that** the concentration of compounds of formula (I) is between 0.001% and 3% by weight with respect to the whole of the composition.
